# EUROPEAN PATENT SPECIFICATION

(11) **EP 0 594 847 B1**
(45) Date of publication and mention of the grant of the patent: **23.12.1998**
(21) Application number: 91912352.1
(22) Date of filing: 11.07.1991
(51) Int. Cl.: C12N 15/61, C12N 9/90, C12N 1/21

(54) **DNA CODING FOR HUMAN FK506-BINDING PROTEIN AND EXPRESSION THEREOF**
FÜR MENSCHLICHEN FK506-BINDENDES PROTEIN KODIERENDE DNS UND DEREN EXPRESSION
ADN CODANT POUR UNE PROTEINE DE LIAISON DE FK506 HUMAINE ET EXPRESSION DE CET ADN

(30) Priority: 11.07.1990 JP 183238/90
(43) Date of publication of application: 04.05.1994
(73) Proprietor: Tonen Corporation, Tokyo 100 (JP)
(72) Inventor: MAKI, Noboru, Tonen Corporation Sougoukenkyusho, Iruma-gun, Saitama-ken 354 (JP); TAKAHASHI, Nobuhiro, Tonen Corp. Sougoukenkyusho, Iruma-gun, Saitama-ken 354 (JP); SUZUKI, Masanori, Tonen Corp. Sougoukenkyusho, Iruma-gun, Saitama-ken 354 (JP)
(74) Representative: Woods, Geoffrey Corlett
(86) International application number: JP9100931
(87) International publication number: WO9201052

(56) References cited:
- EP-A- 481 673
- EP-A- 0 379 342
- WO-A-91/04321
- NATURE vol. 346, no. 6285, 16 August 1990, MACMILLAN JOURNALS LTD.,LONDON, UK; pages 671 - 674 R.F. STANDAERT ET AL. 'Molecular cloning and overexpression of the human FK506-binding protein FKBP'
- Proc. Natl. Acad. Sci. USA, Vol. 87, No. 14, 1990, MAKI N. et al., "Complementary DNA encoding the human T-cell FK-506-binding protein, a peptidylprolyl cis-trans isomerase distinct from cyclophilin", pages 5440-5443.
- SAMBROOK et al., "Molecular Cloning", 1989, Cold Spring Harbor Laboratory Press, Section 16-17.
- Nature, Vol. 341, 1989, SIEKIERKA J. et al., "A cytosolic binding protein for the immunosuppressant FK506 has peptidylprolyl isomerase activity but is distinct from cyclophilin", pages 755-757.
- Nature, Vol. 341, 1989, HARDING M. et al., "Areceptor for the immunosuppressant FK506 isa cis-trans peptidyl-prolyl isomerase", pages 758-760.

## Description

This invention relates to a DNA molecule coding for human FK506-binding protein and the expression thereof.

### Background of the Invention

Peptidyl prolyl cis-trans isomerase (referred to as "PPIase" hereinafter) is known as the enzyme which catalyzes isomerization of a proline-containing peptide linkage and promotes formation of the higher-order structure of a protein. This enzyme has been proved to be identical to cyclophilin which binds specifically to an immunosuppressive agent, cyclosporin A, (Takahashi, N. *et al., Nature,* vol.337, pp.473 - 475, 1989; Lang, K. *et al., Nature,* vol.329, pp.268 - 270, 1987). PPIase is also capable of promoting folding of the higher-order structure of denatured proteins such as ribonuclease A, ribonuclease T₁, immunogloblin, cytochrome C and type III collagen (Fischer, G. and Bang, H., *Biochimi. Biopys. Acta,* vol.828, pp.39 - 42, 1985; Lin, L.-N *et al., Biochimi. Biophys. Acta,* vol.956, pp.256 - 266, 1989; Bachinger, H.P., *J. Biol. Chem.,* vol.262, pp.17144 - 17148, 1987).

From the fact that PPIase activity of cyclophilin is inhibited by the immunosuppressive agent cyclosporin A, such an effect of cyclosporin A has been considered to be achieved by inhibiting the activity of PPIase and thus repressing exhibition of the activity of a substrate protein for PPIase. However, as described below, it has been suggested that there exist a number of protein species as substrate for cyclophilin, as well as a variety of effects of cyclosporin A. For example, cyclosporin A activates the helper T cell among cells involved in the immune system, and cyclophilin serves as PPIase and is involved in the activation of certain transcriptional control factors, such as NFAT and AP-3, which induce expression of interleukin-2 gene and other lymphokine genes (Takahashi N. *et al*, *Gendai Kagaku* (Japan), vol.222, pp.40 - 47, 1989; Emmel, E.A. *et al, Science,* vol.246, pp.1617 - 1620, 1989). Also, cyclosporin A inhibits production and secretion of γ-interferon, interleukin-4, macrophage-activating factor, tumor necrosis factor and the like in immune cells, and cyclophilin probably serves as PPIase in the transcription, translation, or formation of higher-order structures of those proteins (Takahashi N., *Igaku No Ayumi* (Japan), vol.151, pp.413 - 416, 1989).

In mammals, cyclophilin has been identified as being one type of protein per biospecies. However, various types of cyclophilin-like proteins are now believed to exist in mammals because it has been found that there are several tens of DNA copies encoding cyclophilin-like proteins on mammalian chromosomal DNA (Haendler, B. *et al., EMBO J.,* vol.6, pp.947 - 950, 1987) and because two different types of cyclophilin-like proteins have been found in yeast and *Escherichia coli* (Japanese Patent Application No. 1-184738 and Japanese Patent Application No. 1-344705). Such a diversity is correlated with a variety of the related substrate proteins, and this may be the cause of the side effects of cyclosporin A when used as a drug.

FK506 is also known as the immunosuppressive agent which has quite similar effects to those of cyclosporin A but has a completely different chemical structure (Thomson, A.W., *Immunology Today,* vol.10, pp.6 - 9, 1989). In addition, it is known that a binding protein having the specific affinity to FK506 in vivo exists and that the FK506-binding protein has PPIase activity which is inhibited by the binding of FK506 (Siekierka, J.J. *et al., Nature,* vol.341, pp.755 - 757, 1989; Harding, M.W. *et al, Nature,* vol.341, pp.758 - 760, 1989).

The fact that these two immunosuppressive agents, i.e., cyclosporin A and FK506, have the same function of inhibiting PPIase activity, in spite of their completely different chemical structures, strongly suggests that immunosuppression is mediated through the inhibition of PPIase activity. In other words, any substance capable of inhibiting PPIase activity may possess immunosuppressive activity.

From the comparative studies on the substrate specificities of FK506-binding protein and cyclophilin based upon their PPIase activities, it has been found that cyclophilin has a relatively wide range of substrate specificity while FK506-binding protein has a narrower range of specificity (Harrison, R.K. and Stein, R.L., *Biochemistry,* vol.29, pp.3813 - 3816, 1990). These findings suggest that substrates for FK506-binding protein may be different from those for cyclophilin, and FK506-binding protein acts with higher specificity compared with cyclophilin.

An immunosuppressive agent that can act selectively to immune-system cells and, especially, can specifically inhibit PPIase activity in helper T cells is expected to be a useful active agent having fewer side effects, and any immunosuppressive agent having few or fewer side effects can be screened for based upon the activity of inhibiting FK506-binding protein which has a narrower range of substrate specificity than cyclophilin.

FK506-binding protein has been isolated directly from natural sources by two research groups, but the reported molecular weights thereof are different from each other: 11,000 daltons as reported by Siekierka, J.J. *et al.* (Nature, vol.341, pp.755 - 757, 1989) and 14,000 daltons as reported by Harding, M.W. *et al.* (*Nature*, vol.341, pp.758 - 760, 1989). In the latter reference, Harding *et al.* have determined the partial amino acid sequence (up to 40 residues of the amino terminal region) of the protein from bovine thymus, and concluded that the isolated protein is different from cyclophilin, though it is not clear whether the protein has any commonness with cyclophilin in terms of the active site of PPIase.

As aforesaid, the FK506-binding protein has a different PPIase activity from that of cyclophilin and it seems to have various physiological functions. This binding protein is regarded as an important material which will be used as a means for screening any immunosuppressive agent based on the inhibition of its PPIase activity, and as a means for promoting in vitro formation of the higher-order structure of a specific protein, as well as its use as a reagent for the examination of substrate specificity of the binding protein. A process for the manufacture of FK506-binding protein, however, has not been established except for its direct isolation from mammalian organs.

Accordingly, an object of the present invention is to determine a DNA sequence coding for the FK506-binding protein and an amino acid sequence deduced from the DNA sequence, by isolating the gene that encodes the binding protein.

Another object of the present invention is to provide a process for manufacturing the FK506-binding protein on a large scale using genetic engineering techniques.

### Summary of the Invention

The present invention provides a DNA molecule comprising a base sequence coding for the human FK506-binding protein which is represented by the amino acid sequence of position 1 - position 108 of SEQ ID NO:1.

A gene encoding the human FK506-binding protein can be obtained by plaque hybridization from a human T cell cDNA library supplied from CLONTECH Inc. (U.S.A.), which has been prepared using λgt 11 as a vector. The molecular weight of the human FK506-binding protein is 11,951 daltons as calculated based on its deduced amino acid sequence, and its isoelectric point is 8.71. The nucleotide sequence of this gene is completely novel compared to the registered data bases and has no homology with that of cyclophilin having a PPIase activity.

The present invention also provides an expression vector containing the DNA molecule and a transformant obtained by introducing the expression vector into a host cell.

In addition, the present invention provides a process for the manufacture of a recombinant human FK506-binding protein, which comprises expressing the vector through a culture of the transformant, as well as the recombinant human FK506-binding protein obtained.

### Brief Description of the Drawings

Fig. 1 shows the construction of the plasmid pUC-X-FKBP-A which contains a DNA fragment coding for the human FK506-binding protein.

Fig. 2 shows the construction of the plasmid pJDB-GAP-FKBP-A.

### Detailed Description of the Invention

The present invention provides a DNA molecule comprising a base sequence coding for human FK506-binding protein which is represented by the amino acid sequence of position 1-position 108 of SEQ ID NO:1.

The following describe isolation, sequencing, and expression of the cDNA concerning this invention:

### Isolation and sequencing of cDNA

A probe to be used can be synthesized based on the amino acid sequence (from Glu at position 31 to Asp at position 37) of bovine FK506-binding protein (referred to as "FKBP" hereinafter) which has been reported by Harding *et al. (Nature,* vol.341, pp.758 - 760, 1989). Using the synthetic probe, a positive clone containing human FKBP cDNA, which has been transfected into an *Escherichia coli* strain, can be screened by hybridization from the human T cell cDNA library which has been prepared by CLONTECH Inc.(U.S.A.), using λgt 11 as a vector.

The positive clone is lysed to obtain phage particles from which phage DNA is recovered by conventional phenol/chloroform extraction and ethanol precipitation. After digesting the phage DNA with *Eco*RI in a buffer solution containing RNase A, the resulting fragments are subjected to agarose gel electrophoresis and Southern blotting (Southern, E.J., *J. Mol. Biol.,* vol.98, pp.503 - 517, 1975). A clone containing an *Eco*RI fragment of about 1.5 kb in length is obtained by repeating the hybridization step, and the EcoRI fragment is isolated and purified from the agarose gel by a conventional technique such as the glass powder method (Gene Clean™, Bio-101).

Next, the 1.5 kb *Eco*RI fragment obtained is inserted into an EcoRI-digested vector pUC 19 using T4 DNA ligase in order to construct a recombinant plasmid. The recombinant plasmid is then transferred into a competent *E. coli* JM 107 strain by the calcium chloride method (Mandel, M. and Higa, A., *J. Mol. Biol.,* vol.53, pp.159 - 162, 1979). A transformant, pUC-h-FKBP, can be obtained by screening ampicillin resistant colonies from the transformed cells. Insertion of the 1.5 kb *Eco*RI fragment into the pUC vector can be confirmed by performing the Mini-Prep of plasmid DNA in the standard way (Maniatis *et al.,* Molecular Cloning: A Laboratory Manual, 1982), digesting the plasmid DNA with *Eco*RI*,* and then subjecting the resulting fragments to agarose gel electrophoresis. The binding ability of the fragment to the probe can also be ascertained by Southern blotting.

The plasmid DNA obtained is double-digested with *Eco*RI and *Hinc*II at the *Hinc*II recognition site located in the DNA insert, and each of the two DNA fragments formed (about 750 bp each) is re-inserted into M13mp phage DNA to determine their nucleotide sequences by dideoxy method (Sanger, F., Nicklen, S. and Corlson, A.R., *Proc. Natl. Acad. Sci.* USA, vol.74, pp.5463 - 5467, 1977). In this way, cDNA which encodes human FKBP can be identified.

Both the nucleotide sequence of the full length human FKBP cDNA and the deduced amino acid sequence are shown in SEQ ID NO:1 of the Sequence Listing. This gene encodes the protein consisting of 108 amino acid residues (¹Met Gly ··· ··· Leu ¹⁰⁸Glu), as demonstrated by the fact that the above amino acid sequence of human FKBP coincides with 40 amino acid residues from the N-terminus of bovine FKBP which have been reported by Harding *et al. (Nature,* vol.341, pp.758 - 760, 1989).

According to an embodiment of the present invention, a base sequence coding for the human FK506-binding protein includes the nucleotide sequence of position 79 - position 402 (⁷⁹ATG GGA ··· ··· CTG ⁴⁰²GAA) shown in SEQ ID NO:1. Any other base sequence, based on the degeneracy of genetic code, which comprises different codons for the same amino acids is also included within the scope of the present invention.

### Characteristics of human FKBP

The molecular weight and isoelectric point of human FKBP are 11,951 daltons and 8.71, respectively, as calculated from the deduced amino acid sequence. These data coincide with the molecular weight reported by Harding *et al. (Nature* Vol.341, pp.758 - 760, 1989) and with the isoelectric point by Siekierka *et al. (Nature,* vol.341, pp.755 - 757, 1989), and thus this indicates that the gene obtained encodes human FKBP. The base and amino acid sequences determined were compared with those registered in data bases (Gen Bank, EMBL, DDBJ), but no homologous gene or protein could be found. Also, there was no homology between human FKBP and cyclophilin when compared to the sequence of cyclophilin which has a PPIase activity (Example 2 herein and Haendler et *al., ENBO J.,* vol.6, pp.947 - 950, 1987). This reveals therefore that the structure of FKBP is completely different from that of cyclophilin. Such a structural difference indicates that these two PPIases probably have different substrate specificity from each other.

When the existence of FKBP-specific mRNA in some organs including lungs, placenta, liver and brain, and in leucocytes was examined by Northern blotting using the isolated gene as a probe, the mRNA was found in all organs and leucocytes tested though the mRNA's content was different among them. Therefore, this means that FKBP is not present tissue-specifically in mammals. When the number of copies of the particular genes on human chromosomes was examined by Southern blotting, only 1 or 2 copies were detected for the FKBP gene while 20 or more copies have been reported for the cyclophilin gene. These results show that cyclophilin has a variety of molecular species, while the function of FKBP is markedly limited because FKBP has no such variety. Thus, protein substrates on which FKBP can act are presumed to be relatively limited, because in respect to FKBP the number of molecular species is small and the range of the substrate specificity based upon PPIase activity is very narrow (Harrison, R.K. and Stein, R.L., *Biochemistry*, vol.29, pp.3813 - 3816, 1990). If the protein substrate of FKBP has a tissue-specific function, a substance which can inhibit the FKBP activity probably works tissue-specifically when used as a drug, whereas the inhibitor as an active agent may often give a side effect if it is a common and ubiquitous protein in mammalian tissues. Therefore, if FKBP is easily available, not only can this protein be used as a useful means for screening for a substrate of FKBP, but also it will have an important role in the screening of FKBP inhibitors such as an immunosuppressive agent having negligible or less side effect.

### Expression of human FK506-binding protein

The present invention also provides an expression vector containing the DNA molecule which comprises a base sequence coding for the human FKBP represented by the amino acid sequence of position 1 - position 108 of SEQ ID NO:1, said DNA molecule being introduced into a cloning site downstream from a promoter and upstream from a terminator in the vector.

### Construction of expression vector:

Any known plasmid or phage may be used as a vector. The promoter to be used may be selected from various promoters originated from *E. coli,* phages, eukaryotes such as yeast, and the like. The exemplified promoters include tryptophan operon (*trp*), lactose operon (*lac*)*,* lambda phage P_{L} or P_{R}, glyceraldehyde-3-phosphate dehydrogenase (GAPDH), alcohol dehydrogenase I (ADH I) promoters, and the like. Examples of the terminator are terminators of the GAPDH gene and the ADH I gene.

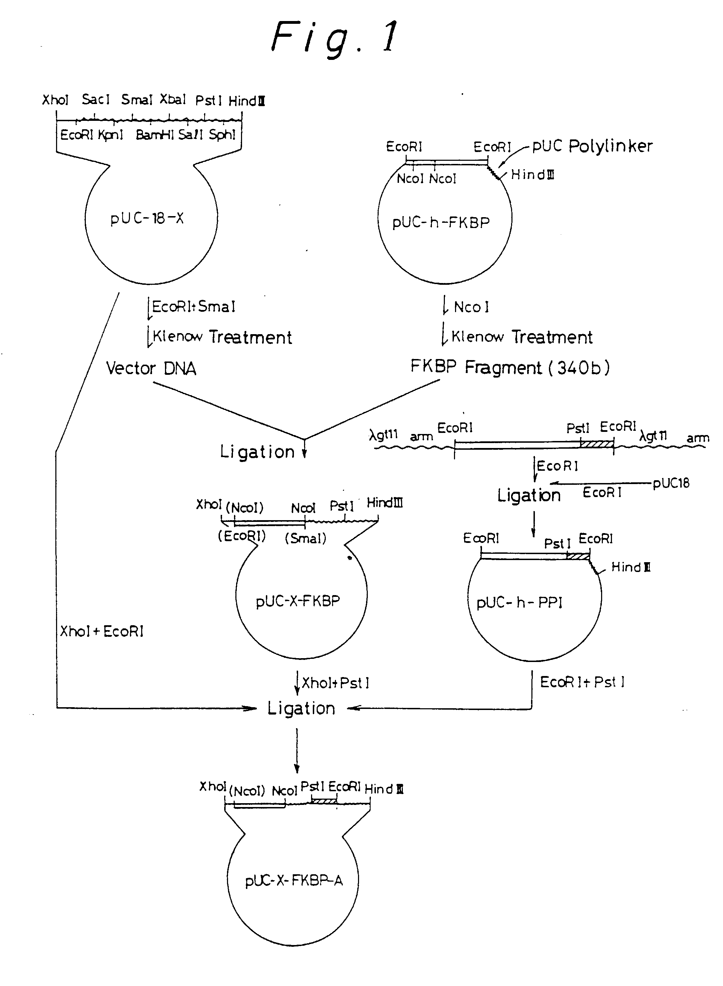

Since the human FKBP clone prepared as aforesaid does not contain a poly A sequence at its 3' end, the plasmid in which the FKBP structural gene is ligated with a poly A sequence from human cyclophilin cDNA is firstly constructed in accordance with the procedure shown in Fig. 1.

The plasmid pUC-h-FKBP DNA containing FKBP cDNA is digested with *Nco*I and then subjected to treatment with Klenow fragment to obtain a 340 bp DNA fragment in which the *Nco*I site is blunt-ended. The DNA fragment obtained is purified using Gene Clean™. Separately from this, the cloning vector pUC-18-X in which an *Xho*I site is introduced into the EcoRI site of pUC 18 is double-digested with EcoRI and *Sma*I and then subjected to the treatment with Klenow fragment to obtain a vector having blunt ends. Thereafter, the vector obtained is ligated with the 340 bp DNA fragment in the presence of T4 DNA ligase to isolate the recombinant plasmid pUC-X-FKBP.

The plasmid pUC-h-PPI DNA containing human PPIase gene is double-digested with *Eco*RI and *Pst*I to obtain a DNA fragment containing a human PPIase poly A sequence of about 150 bp.

The pUC-18-X DNA is also double-digested with EcoRI and *Xho*I to obtain a vector fragment of 2.6 kb.

These three DNA fragments prepared above are subsequently ligated together to construct the plasmid pUC-X-FKBP-A (Fig. 1).

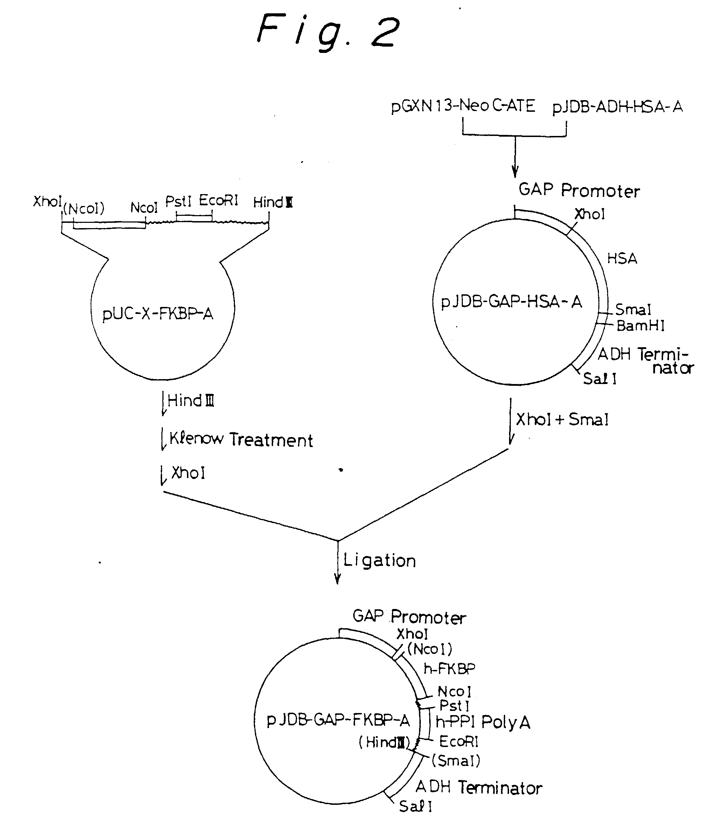

In addition, the plasmid containing a promoter region of natural GAPDH gene (pGXN13-NeoC-ATE; Japanese Patent Application No. 1-328264) is double-digested with *Xho*I and *Bam*HI, and a large fragment obtained is ligated with a smaller fragment (containing HSA cDNA) of *Xho*I*/Bam*HI double digests of the expression plasmid pJDB-ADH-HSA-A which contains human serum albumin cDNA (see Japanese Patent Application Laying-Open (KOKAI) No. 2-117384). The resulting pJDB-GAP-HSA-A is then double-digested with *Sma*I and *Xho*I to obtain an expression vector from which the HSA gene is removed. Separately from this, pUC-X-FKBP-A is in turn subjected to *Hind*III digestion, treatment with Klenow fragment and *Xho*I digestion to obtain a 570 bp DNA fragment containing FKBP and poly A sequences. By ligating these DNA fragments with each other, the expression plasmid pJDB-GAP-FKBP-A is constructed for the expression and production of human FKBP (see Fig. 2). As illustrated by the structure of the expression plasmid in Fig. 2, a DNA fragment encoding human FKBP is introduced into a site downstream from the GAPDH promoter and upstream from the ADH I terminator. The expression plasmid pJDB-GAP-FKBP-A has been transferred into *E. coli* strain HB101, and the transformant obtained has been deposited on June 27, 1990 with Fermentation Research Institute, Agency of Industrial Science and Technology, 1-3 Higashi 1-chome, Tsukuba-shi, Ibaraki-ken, 305, Japan, as the transformant HB101/pJDB-GAP-h-FKBP (Accession Number: FERM P-11558). This deposition has been subsequently converted on July 4, 1991 to an international deposition under the Budapest Treaty by the same depositary institution as an international depositary authority set forth in the Budapest Treaty to be given new Accession Number: FERM BP-3471.

### Preparation of transformant:

The present invention also provides a transformant obtained by transforming a host with the expression vector of this invention.

The transformant may be prepared by incorporating the expression plasmid pJDB-GAP-FKBP-A into a host by a modification of the KUR method of Hashimoto and Kimura *(Hakko To Kogyo* (Japan), vol.43, pp.630 - 637, 1985). Various microorganisms generally used in the art, for example, *E. coli, Bacillus subtilis* and yeast, can be employed as a host in the transformation step. It is apparent to those skilled in the art that animal cells can also be employed as host.

### Production of recombinant human FKBP:

Recombinant human FKBP can be obtained by culturing the transformant in an appropriate medium to produce and accumulate the protein. Such a process comprises the steps of:
constructing an expression vector capable of
replicating and of expressing a DNA molecule containing the base sequence coding for the amino acid sequence of position 1 - position 108 of SEQ ID NO:1 in an appropriate host;
incorporating said expression vector into said host to produce transformant cells;
culturing said transformant cells under such conditions that said DNA is expressed to produce said recombinant human FKBP; and
recovering said recombinant human FKBP.

The process for the manufacture of recombinant human FKBP is also included within the scope of the present invention.

Detection of the expressed product was carried out by collecting cells from the culture, lysing the cells by heating and analyzing the lysed sample by SDS-polyacrylamide gel electrophoresis, and thereby a single band characterized as human FKBP was detected at about 14 kilodaltons on the gel. The expressed product can be recovered in a substantially purified form, by disrupting the cells and then subjecting the extracts obtained to conventional purification means, for example, a combination of gel filtration, ion exchange chromatography and affinity chromatography. Accordingly, the present invention is also directed to the recombinant human FKBP obtained by the process.

According to the present invention, it is possible to produce the recombinant human FKBP on a large scale using genetic engineering techniques, and the FKBP having PPIase activity will be supplied for the screening of immunosuppressive agents. In addition, the recombinant protein of the present invention may be utilized for activation of an inactive protein, because the recombinant protein is expected to promote formation of the higher-order structure of a different substrate protein from that of cyclophilin.

The present invention is illustrated in further detail by the following examples, but it should be understood to those skilled in the art that the present invention is not limited by the examples.

### Examples

### Example 1 Cloning of human FKBP gene

A human T cell cDNA library, which has been prepared using λ gt11 as a vector, supplied from CLONTECH Inc.(U.S.A.) was used to obtain a clone containing human FKBP cDNA.

1 x 10⁴ pfu of λ gt11 recombinant phages were added to 50 µl of the culture of *E*. *coli* strain Y1090 which had been precultured overnight in LB medium (1% tryptone, 0.5% yeast extracts, 0.5% NaCl) supplemented with 0.2% maltose, and the mixture was incubated at 37°C for 20 minutes. The resulting incubation mixture was mixed with 3 ml of L-Top-agarose (LB medium plus 0.7% agarose) and seeded on four L-plates (LB medium; 1.5% agar) of 90 mm in diameter. All the plates were cultured overnight at 37°C to form plaques and then maintained at 4°C for 1 hour. Recombinant phage particles were transferred to a membrane filter (Hybond-N, Amersham), and the filter was placed for 5 minutes on a 3MM filter paper (Whatman), which had been pre-soaked in a solution containing 0.5 N NaOH and 1 M NaCl, and then for a further 5 minutes on another filter paper pre-soaked in 0.5 M Tris-HCl (pH 7.2) plus 1.5 M NaCl. The thus-treated membrane filter was washed with 2 x SSC (20 x SSC consists of 3 M NaCl and 0.3 M trisodium citrate), air-dried, covered with a resin film and then exposed to UV rays in order to fix phage DNA on the filter. Thereafter, the membrane filter was subjected to screening using a ³²P-labeled synthetic oligonucleotide (specific activity, ≥ 10⁷ cpm/µg DNA) as a probe (Benton and Davis, *Science,* vol.196, pp.180 - 182, 1977).

The probe DNA fragment 5'-GA(A/G)GA(T/C)GG(G/A/T/C)AA(A/G)AA(A/G)TT(T/C)GA-3' for hybridization, which corresponds to a sequence of from glutamic acid at position 31 to aspartic acid at position 37 in the amino acid sequence of bovine FKBP (Harding *et al., Nature,* vol.341, pp.758 - 760, 1989), was synthesized using the automatic DNA synthesizer (Model 380B, Applied Biosystems) with the principle of the phosphoamidite method developed by Caruthers *et al.* (Matteucci, M.D. and Caruthers, M.H., *Tetrahedron Letters,* vol.21, p.719, 1980). The 5' end the DNA chain synthesized (21 pmol) was phosphorylated by treatment at 37°C for 60 minutes in 50 µl of the solution consisting of 50 mM Tris-HCl (pH 7.6), 10 mM MgCl₂, 5 mM dithiothreitol, 100 µCi of [γ-³²P] ATP (3,000 Ci/mmol, Amersham) and 12 units of T4 polynucleotide kinase (Takara Shuzo Co., Ltd.). The membrane filter prepared above was subjected to hybridization at 37°C for 16 hours in the solution containing 6 x SSC, 5 x Denhardt's solution (100 x Denhardt's solution consists of 2% bovine serum albumin, 2% Ficoll and 2% polyvinyl pyrrolidone), 0.5% SDS, 50 µg/ml of ultrasonic-treated salmon sperm DNA, and 10⁶ cpm/ml of said probe DNA. The filter was washed with 2 x SSC at 37°C and then exposed to X-ray at -70°C for 10 hours overlaid with an X-ray film (XAR-5, Kodak).

After development of the film, each of the two plaques which showed positive signals was scratched off using the tip of a Pasteur pipet and the phage particles obtained were suspended in 100 µl of TM solution [10 mM Tris-HCl (pH 7.5), 10 mM MgCl₂]. After standing still at room temperature for 20 minutes, 0.5 µl of the suspension was diluted with 1 ml of TM solution. *E. coli* Y1090 cells were infected with 5 µl of the suspension diluted, seeded on L-plate, and cultured in the same manner as above in order to form plaques. By subjecting the plaques formed to plaque hybridization in the same manner as described above, a positive clone forming a single plaque was harvested. The positive clone was scratched off using the tip of a Pasteur pipet, added to 50 µl of the suspension of *E. coli* strain Y1090 and maintained at 37°C for 20 minutes. The resulting mixture was then added to 2 ml of LB medium containing 10 mM MgSO₄ and cultured at 37°C for 6 hours with shaking. The culture obtained was mixed with 100 µl of chloroform, stirred on a Vortex mixer to lyse the cells completely and then subjected to centrifugation at 5,000 rpm for 5 minutes. The supernatant contained phage particles of the order of 10¹⁰. The supernatant of 800 µl was mixed thoroughly with 100 µl of 5 M NaCl and stood for 20 minutes at -20°C. After centrifuging the mixture at 15,000 rpm for 5 minutes, the pellet obtained was washed with 500 µl of 70% ethanol and then dissolved in 200 µl of TE solution [10 mM Tris-HCl (pH 8.0), 1 mM EDTA). The resulting solution was mixed with 1 µl (60 units/µl) of DNase I (Takara Shuzo Co., Ltd.) and 2 µl of 1 M MgCl₂, incubated at 37°C for 30 minutes, and then mixed with 100 µl of TE-saturated phenol on a Vortex mixer. The mixture treated was centrifuged at 12,000 rpm for 5 minutes, and the aqueous layer was extracted once with phenol/chloroform (1:1). To the aqueous layer after extraction were added 20 µl of 3 M sodium acetate (pH 5.2) and 500 µl of ethanol, and the mixture was then centrifuged to precipitate the DNA. The precipitate was washed with 70% ethanol, dried *in vacuo* and then dissolved in 50 µl of TE solution. In this manner, about 1 µg of phage DNA was obtained. To 20 µl of the prepared solution were added 2.5 µl of 10 times-concentrated *Eco*RI buffer [0.5 M NaCl, 1 M Tris-HCl (pH 7.5), 70 mM MgCl₂], 1 µl (20 units) of *Eco*RI (Nippon Gene), and 1 µl of 10 mg/ml solution of RNase A (Sigma). After incubating at 37°C for 1 hour, the reaction mixture was subjected to 0.7% agarose gel electrophoresis, and the DNA bands were transferred to a Hybond filter according to the Southern blotting (Southern, E., *J. Mol. Biol.,* vol.98, pp.503 - 517, 1975). Thereafter, hybridization on the DNA-bound filter was carried out under the same conditions as in the plaque hybridization. Each of the clones thus obtained contained an EcoRI fragment of about 1.5 kilo bases which was then isolated from agarose gel by glass powder method (Gene Clean™, Bio-101) and subcloned into pUC19 vector.

The *Eco*RI-digested pUC19 vector (30 ng) and the 1.5 kb *Eco*RI fragment (20 ng) recovered were treated at 16°C for 4 hours in 30 µl of the reaction mixture [66 mM Tris-HCl (pH 7.6), 6.6 mM MgCl₂, 10 mM dithiothreitol, 1 mM ATP] containing 2.8 units of T4 DNA ligase (Takara Shuzo Co., Ltd.) so as to obtain a recombinant plasmid in which the digested vector and the 1.5 kb fragment were ligated together. This reaction mixture of 10 µl was used to transform *E. coli* strain JM107 as a host. A competent *E. coli* strain for transformation was prepared by the calcium chloride method (Mandel, M. and Higa, A., *J. Mol. Biol.,* vol.53, pp.159-162, 1970). An overnight culture (in LB medium) of *E. coli* strain JM107 was diluted with the same medium and cultured at 37°C with shaking until absorbance at OD₆₆₀ reached 0.6. The culture (1.5 ml) was centrifuged at 5,000 rpm for 5 minutes. The cells collected were suspended in 750 µl of 50 mM CaCl₂ solution, maintained for 20 minutes on ice, and then centrifuged to collect cells. The cells were again suspended in 100 µl of 50 mM CaCl₂ solution, and the cell suspension was mixed with the aforementioned DNA ligase reaction mixture and then stood for 40 minutes on ice. After keeping at 42°C for 1 minute, the resulting mixture was mixed with 1 ml of LB medium and incubated at 37°C for 30 minutes. The mixture (0.1 ml) incubated was applied on an X-Gal plate (L-plate supplemented with 155 µg/ml of 5-bromo-4-chloro-3-indolyl-β-D-galactopyranoside, 80 µg/ml of isopropyl-β-D-thiogalactopyranoside and 25 µg/ml of ampicillin). After culturing overnight at 37°C, white colonies were selected from colonies formed on the plate and a loopful of cells of the selected colony was transferred to LB medium supplemented with 25 µg/ml of ampicillin in order to prepare an overnight culture. Cells were collected from 1.5 ml of the overnight culture by centrifugation and then subjected to a Mini-Prep of plasmid DNA in the conventional way (Maniatis *et al., Molecular Cloning: A Laboratory Manual*, 1982). Plasmid DNA obtained was digested with *Eco*RI and the resulting fragments were subjected to agarose gel electrophoresis to confirm the existence of the 1.5 kb *Eco*RI fragment inserted into the pUC vector (pUC-h-FKBP). Binding of the insert to the probe was also confirmed by Southern blotting. At the *Hind*II recognition site located in the DNA insert, 1 µg of the plasmid DNA was double-digested at 37°C for 60 minutes in 25 µl of the reaction mixture [50 mM NaCl, 100 mM Tris-HCl (pH 7.5), 7 mM MgCl₂, 20 units of EcoRI (Nippon Gene) and 10 units of *Hind*II (Nippon Gene)]. Each of the two DNA fragments (about 750 bp each) formed was inserted into M13mp phage DNA so as to determine their nucleotide sequences by the dideoxy method (Sanger, F., Nicklen, S. and Corlson, A.R., *Proc. Natl. Acad. Sci.* USA, vol.74, pp.5463 - 5467, 1977). In this way, the cDNA which encodes human FKBP was identified. The determined nucleotide sequence of the 1.5 kb *Eco*RI fragment is shown in SEQ ID NO:1 of Sequence Listing. This fragment consists of 1532 base pairs. By comparing the deduced amino acid sequence from the base sequence with the partial amino acid sequence of bovine FKBP reported by Harding *et al. (Nature,* vol.341, pp.758 - 760, 1989), it was revealed that this inventive gene encodes human FKBP consisting of 108 amino acids of from position 1 to position 108 represented by SEQ ID NO:1.

### Example 2 Cloning of human cyclophilin cDNA

A human T cell cDNA library, which has been prepared using λ gt11 as a vector, supplied from CLONTECH Inc. (U.S.A.) was used to obtain a clone which contained human cyclophilin cDNA. *E. coli* strain Y1090 as a host was infected with λ gtll recombinant phages and thus 1 x 10⁴ plaques were formed on an L-plate, and the recombinant DNA fragments obtained were transferred to a membrane filter (Hybond-N, Amersham). The membrane filter was then screened using a ³²P-labeled porcine PPIase cDNA gene (specific activity, ≥ 10⁸ cpm/µg) as a probe (Benton and Davis, *Science,* vol.196, pp.180 - 182, 1977).

The probe was prepared by labeling the *Eco*RI*/Ps*tI fragment of the porcine PPIase gene as disclosed in Japanese Patent Application No. 1-184738 (filed July 19, 1989) using a randomly primed DNA labeling kit (Boehringer) according to the supplier's manual attached to the kit. The membrane filter prepared above was subjected to hybridization at 65°C for 16 hours in the solution consisting of 4 x SSC, 5 x Denhardt's solution, 0.5% SDS, 50 µg/ml of ultrasonic-treated salmon sperm DNA and 10⁶ cpm/ml of the probe DNA. Thereafter, the filter was washed with 0.1 x SSC at 65°C and then exposed to X-ray for 10 hours at -70°C after it had been overlaid with an X-ray film (XAR-5, Kodak). After development of the film, the plaque which showed a positive signal was subjected repeatedly to the hybridization step until a positive clone was obtained as a single plaque.

Phage DNA was prepared from the positive clone (Blattner *et al., Science*, vol.202, pp.1279 - 1284, 1978), and 1 µg of the phage DNA was digested at 37°C for 60 minutes in 25 µl of the reaction mixture [50 mM NaCl, 100 mM Tris-HCl (pH 7.5), 7 mM MgCl₂ and 20 units of EcoRI (Nippon Gene)]. The resulting digests were subjected to Southern blotting (Southern, E., *J. Mol. Biol.,* vol.98, pp.503 - 517, 1975) and the blots on filter were hybridized with the probe. The hybridized EcoRI fragment of about 750 bp was recovered from the agarose gel by the glass powder method (Gene Clean™, Bio-101) and subcloned into pUC19 vector.

*Eco*RI-digested pUC19 vector (30 ng) and the *EcoRI* fragment (determined to be 800 bp) (20 ng) recovered were treated at 16°C for 4 hours in 30 µl of the reaction mixture [66 mM Tris-HCl (pH 7.6), 6.6 mM MgCl2, 10 mM dithiothreitol, 1 mM ATP] containing 2.8 units of T4 DNA ligase (Takara Shuzo Co., Ltd.) to obtain a recombinant plasmid in which the digested vector and the 800 bp fragment were ligated together. *E. coli* strain JM107 was transformed with 10 µl of the reaction mixture obtained, and the transformant was inoculated on the X-Gal plate in order to form colonies. A white colony was selected and then inoculated in 5 ml of LB medium containing 25 µg/ml of ampicillin. After culturing overnight at 37°C, cells were collected from 1.5 ml of the overnight culture by centrifugation and subjected to the Mini-Prep so as to prepare DNA. After cleavage of the DNA with *Eco*RI, the fragments were subjected to agarose gel electrophoresis to obtain the recombinant plasmid pUC-h-PPI containing an appropriate DNA insert.

At the *Pst*I recognition site located in the DNA insert, 1 µg of the plasmid DNA was double-digested at 37°C for 60 minutes in 25 µl of the solution consisting of 50 mM NaCl, 100 mM Tris-HCl (pH 7.5), 7 mM MgCl₂, 20 units of *EcoRI* (Nippon Gene) and 20 units of *Pst*I (Nippon Gene). About 600 bp- and about 150 bp-DNA fragments formed were separately inserted into a M13mp phage DNA in order to determine their nucleotide sequences by the dideoxy method (Sanger, F., Nicklen, S. and Corlson, A.R., *Proc. Natl. Acad. Sci.* USA, vol.74, pp.5463 - 5467, 1977). The cDNA sequence of human cyclophilin has already been determined and reported (Haendler, B *et al., ENBO J.,* vol.6, pp.947 - 950, 1987). The nucleotide sequence of the clone obtained completely coincided with the reported sequence.

### Example 3 Construction of human FKBP expression plasmid for use in yeast

One(1) µg of the plasmid pUC-h-FKBP DNA containing FKBP cDNA was digested in 20 µl of the reaction mixture [150 mM NaCl, 6 mM Tris-HCl (pH 7.5), 6 mM MgCl₂ and 6 units of *Nco*I (Nippon Gene)] at 37°C for 1 hour. The digests were mixed with 80 µl of TE and heated at 70°C for 5 minutes. To the heat-treated sample were added 1 µl of 1 mM dXTP (dATP, dGTP, dCTP or dTTP) and 2 units of the DNA polymerase Klenow fragment (Takara Shuzo Co., Ltd.), and the mixture was incubated at 37°C for 30 minutes. The resulting reaction mixture was subjected to agarose gel electrophoresis, and a 340 bp DNA fragment blunt-ended which has a *Nco*I site was isolated from the gel and purified using Gene Clean™. Separately from this, 1 µg of the cloning vector pUC18X in which a *XhoI* site had been introduced into the EcoRI site of pUC18 was double-digested in 20 µl of the reaction mixture [20 mM KCl, 10 mM Tris-HCl (pH 8.0), 7 mM MgCl₂, 20 units of EcoRI (Nippon Gene) and 10 units of SmaI (Takara Shuzo Co., Ltd.)] at 37°C for 1 hour. The digests were mixed with 20 µl of 40 mM Tris-HCl (pH 7.5) and heated at 70°C for 5 minutes. Thereafter, Klenow treatment was carried out in the same manner as described above to obtain a blunt-ended vector. This vector (30 ng) and the aforementioned FKBP-containing DNA fragment (20 ng) were treated at 16°C for 10 hours in 30 µl of the ligation mixture [66 mM Tris-HCl (pH 7.6), 6.6 mM MgCl₂, 10 mM dithiothreitol and 1 mM ATP] containing 2.8 units of T4 DNA ligase (Takara Shuzo Co., Ltd.) so as to obtain the recombinant plasmid pUC-X-FKBP in which the vector was ligated with the DNA fragment.

To 20 µl of the EcoRI reaction mixture [50 mM NaCl, 100 mM Tris-HCl (pH 7.5), 7 mM MgCl₂ and 20 units of *Eco*RI] was added 1 µl of *Pst*I (20 units, Nippon Gene), and digestion of the plasmid pUC-h-PPI DNA (1µg) containing the human cyclophilin cDNA was carried out in the mixture solution at 37°C for 1 hour to obtain an about 150 bp DNA fragment containing the human cyclophilin poly A sequence. In the same manner, a DNA fragment containing a 370 bp FKBP gene was obtained from the plasmid pUC-X-FKBP by double-digesting 1 µg of the plasmid DNA at 37°C for 1 hour in 20 µl of the *Eco*RI reaction mixture which contains 1 µg of *Xho*I (12 units, Takara Shuzo Co., Ltd.) and 1 µg of *Pst*I (20 units). As a cloning vector, a 2.6 kb-vector fragment was obtained by digesting 200 ng of the pUC18X DNA at 37°C for 1 hour in a mixture mixture of 20 µl of the *Eco*RI reaction mixture and 1 µl of *Xho*I (12 units).

Thereafter, the plasmid pUC-X-FKBP-A was constructed in which these three DNA fragments were ligated together (see. Fig. 1).

Separately from this, the plasmid containing a promoter region of the intact GAPDH gene (pGXN13-NeoC-ATE; Japanese Patent Application No. 1-328624) was double-digested with *Xho*I and *Bam*HI, and a large fragment obtained was ligated with a smaller fragment (containing HSA cDNA) of *Xho*I/*Bam*HI double digests of the expression plasmid pJDB-ADH-HSA-A which contained human serum albumin cDNA (Japanese Patent Application No. 2-117384). One(1) µg of pJDB-GAP-HSA-A DNA thus prepared was then double-digested at 37°C for 1 hour in 20 µl of the reaction mixture [20 mM KCl, 10 mM Tris-HCl (pH 8.0), 7 mM MgCl₂, 10 units of *Sma*I and 12 units of *Xho*I) to obtain an expression vector from which the HSA gene was removed.

From the plasmid pUC-X-FKBP-A, a 570 bp-DNA fragment containing FKBP and poly A sequences were obtained in the following manner: One(1) µg of the plasmid DNA was digested at 37°C for 1 hour in the reaction mixture [50 mM NaCl, 100 mM Tris-HCl (pH 7.5), 7 mM MgCl₂ and 10 units of HindIII (Takara Shuzo Co., Ltd.)]. Thirty µl of water were added to the reaction mixture and heated at 70°C for 5 minutes. The heat-treated sample was subjected to treatment with Klenow fragment and then again to heat treatment. Thereafter, the resulting sample was mixed with 5 µl of 1 M NaCl and 1 µl (12 units) of *Xho*I*,* and the mixture was incubated at 37°C for 1 hour to obtain a 570 bp-DNA fragment.

By ligating these DNA fragments, the expression plasmid pJDB-GAP-FKBP-A was constructed for use in yeast cells (see Fig. 2).

### Example 4 Transformation of yeast host cells with expression plasmid

Transformation of yeast cells with the human FKBP expression plasmid pJDB-GAP-FKBP-A was carried out by a modification of the KUR method reported by Hashimoto and Kimura *(Hakko To Kogyo* (Japan), vol.43, pp.630 - 637, 1985). The yeast strain AH22 (MATa, *leu* 2-3, *leu* 2-112, *his* 4-519, *can*1) was cultured overnight in YPD medium [1% Yeast extracts (Difco Laboratories), 2% Bacto Pepton (Difco Laboratories) and 2% glucose]. One(1) ml of the overnight culture was inoculated in 50 ml of YPD medium and cultured at 30°C until absorbance at OD₆₀₀ reached 0.5. The culture kept at 4°C was then centrifuged at 2,000 rpm for 5 minutes, and the cells collected were resuspended in 5 ml of 0.1 M LiSCN. 1.5 ml aliquot of the cell suspension was centrifuged at 2,000 rpm for 5 minutes and the cells collected were resuspended in the solution consisting of 10 µl of 2 M LiSCN and 46 µl of 50% polyethylene glycol 4000. To the suspension was then added 10 µl of the DNA solution containing the plasmid DNA of 5 to 10 µg, and the mixture was incubated overnight at 30°C. The suspension was mixed with 50 µl of sterile distilled water, stirred gently on a Vortex mixer, and subsequently centrifuged at 2,000 rpm for 5 minutes. The cells collected were resuspended in 100 µl of sterile distilled water and seeded on the selection agar medium [SD medium: 20 µg/ml of histidine hydrochloride, 0.67% amino acid-free Yeast Nitrogen Base (Difco Laboratories) and 2% glucose, supplemented with 2% agar]. Several days after cultivation at 30°C, each colony formed was inoculated in 5 ml of SD medium and cultured at 30°C for 48 hours in order to examine the expression of human FKBP by means of SDS-polyacrylamide gel electrophoresis (SDS-PAGE).

### Example 5 Expression of human FKBP by transformant

One(1) ml of the culture obtained by culturing the transformant in SD medium for 48 hours was centrifuged at 5,000 rpm for 5 minutes, and the cells collected were resuspended in 30 µl of the buffer solution for an SDS-PAGE sample (2% SDS, 5% 2-mercaptoethanol, 7% glycerol, 0.00625% Bromophenol Blue and 0.0625 M Tris-HCl pH 6.8). After boiling the cell suspension at 100°C for 10 minutes, 10 µl of the resulting sample was subjected to electrophoresis on an SDS-polyacrylamide gel with 15% of separation gel concentration (Laemmli's method: *Nature,* vol.277, p.680, 1970). At the end of the electrophoresis, the gel was stained with Coomassie Brilliant Blue (CBB), and a single band corresponding to human FKBP was detected at about 14 kilodaltons of molecular weight.

### Sequence Listing

### SEQ ID NO: 1

SEQUENCE LENGTH: 1532
SEQUENCE TYPE: nucleic acid
STRANDEDNESS: double
TOPOLOGY: linear
MOLECULE TYPE: cDNA to mRNA
IMMEDIATE SOURCE

Human T cell cDNA library (ex CLONTECH Inc., USA) FEATURE
FEATURE KEY: mat peptide
LOCATION: 79 ...... 402
IDENTIFICATION METHOD: S

### SEQUENCE DESCRIPTION

EcoRI
GAATTCGGGC CGCCGCCAGG TCGCTGTTGG TCCACGCCGC CCGTCGCGCC GCCCGCCCGC 60
TCAGCGTCCG CCGCCGCC ATG GGA GTG CAG GTG GAA ACC ATC TCC CCA GGA 111
Met Gly Val Gln Val Glu Thr Ile Ser Pro Gly
1               5                   10
GAC GGG CGC ACC TTC CCC AAG CGC GGC CAG ACC TGC GTG GTG CAC TAC 159
Asp Gly Arg Thr Phe Pro Lys Arg Gly Gln Thr Cys Val Val His Tyr
            15                  20                  25
ACC GGG ATG CTT GAA GAT GGA AAG AAA TTT GAT TCC TCC CGG GAC AGA 207
Thr Gly Met Leu Glu Asp Gly Lys Lys Phe Asp Ser Ser Arg Asp Arg
        30                  35                  40
AAC AAG CCC TTT AAG TTT ATG CTA GGC AAG CAG GAG GTG ATC CGA GGC 255
Asn Lys Pro Phe Lys Phe Met Leu Gly Lys Gln Glu Val Ile Arg Gly
    45                  50                  55
TGG GAA GAA GGG GTT GCC CAG ATG AGT GTG GGT CAG AGA GCC AAA CTG 303
Trp Glu Glu Gly Val Ala Gln Met Ser Val Gly Gln Arg Ala Lys Leu

60                  65                  70                  75
ACT ATA TCT CCA GAT TAT GCC TAT GGT GCC ACT GGG CAC CCA GGC ATC 351
Thr Ile Ser Pro Asp Tyr Ala Tyr Gly Ala Thr Gly His Pro Gly Ile
                80                  85                  90
ATC CCA CCA CAT GCC ACT CTC GTC TTC GAT GTG GAG CTT CTA AAA CTG 399
Ile Pro Pro His Ala Thr Leu Val Phe Asp Val Glu Leu Leu Lys Leu
            95                  100                 105
GAA TGA CA GGAATGGCCT CCTCCCTTAG CTCCCTGTTC TTGGATCTGC CATGGAGGGA 457
Glu
TCTGGTGCCT CCAGACATGT GCACATGAGT CCATATGGAG CTTTTCCTGA TGTTCCACTC 517
CACTTTGTAT AGACATCTGC CCTGACTGAA TGTGTTCTGT CACTCAGCTT TGCTTCCGAC 577
ACCTCTGTTT CCTCTTCCCC TTTCTCCTCG TATGTGTGTT TACCTAAACT ATATGCCATA 637
AACCTCAAGT TATTCATTTT ATTTTGTTTT CATTTTGGGG TGAAGATTCA GTTTCAGTCT 697
TTTGGATATA GGTTTCCAAT TAAGTACATG GTCAAGTATT AACAGCACAA GTGGTAGGTT 757
SspI
AACATTAGAA TAGGAATTGG TGTTGGGGGG GGGGTTTGCA AGAATATTTT ATTTTAATTT 817
TTTGGATGAA ATTTTTATCT ATTATATATT AAACATTCTT GCTGCTGCGC TGCAAAGCCA 877
TAGCAGATTT GAGGCGCTGT TGAGGACTGA ATTACTCTCC AAGTTGAGAG ATGTCTTTGG 937
GTTAAATTAA AAGCCCTACC TAAAACTGAG GTGGGGATGG GGAGAGCCTT TGCCTCCACC 997
ATTCCCACCC ACCCTCCCCT TAAACCCTCT GCCTTTGAAA GTAGATCATG TTCACTGCAA 1057
TGCTGGACAC TACAGGTATC TGTCCCTGGG CCAGCAGGGA CCTCTGAAGC CTTCTTTGTG 1117
ATAACTTTCC AAGCTCCACC ACTTCCTAAA TCTTAAGAAC TTTAATTGAC AGTTTCAATT 1237
GAAGGTGCTG TTTGTAGACT TAACACCCAG TGAAAGCCCA GCCATCATGA CAAATCCTTG 1297
AATGTTCTCT TAAGAAAATG ATGCTGGTCA TCGCAGCTTC AGCATCTCCT GTTTTTTGAT 1357
GCTTGGCTCC CTCTGCTGAT CTCAGTTTCC TGGCTTTTCC TCCCTCAGCC CCTTCTCACC 1417
CCTTTGCTGT CCTGTGTAGT GATTTGGTGA GAAATCGTTG CTGCACCCTT CCCCCAGCAC 1477
EcoRI
CATTTATGAG TCTCAAGTTT TATTATTGCA ATAAAAGTGC TTTATGCCCG AATTC 1532

## Claims

1. A DNA molecule comprising a base sequence coding for human FK506-binding protein which is represented by the following amino acid sequence:

1                   5                             10
Met Gly Val Gln Val Glu Thr Ile Ser Pro

                        15                            20
Gly Asp Gly Arg Thr Phe Pro Lys Arg Gly

                        25                            30
Gln Thr Cys Val Val His Tyr Thr Gly Met

                        35                            40
Leu Glu Asp Gly Lys Lys Phe Asp Ser Ser

                        45                            50
Arg Asp Arg Asn Lys Pro Phe Lys Phe Met

                        55                            60
Leu Gly Lys Gln Glu Val Ile Arg Gly Trp

                        65                            70
Glu Glu Gly Val Ala Gln Met Ser Val Gly

                        75                            80
Gln Arg Ala Lys Leu Thr Ile Ser Pro Asp

85 90
Tyr Ala Tyr Gly Ala Thr Gly His Pro Gly

95 100
Ile Ile Pro Pro His Ala Thr Leu Val Phe

105
Asp Val Glu Leu Leu Lys Leu Glu

2. A DNA molecule according to claim 1 wherein said base sequence comprises the following sequence:

ATG GGA GTG CAG GTG GAA ACC ATC TCC CCA 30

GGA GAC GGG CGC ACC TTC CCC AAG CGC GGC 60

CAG ACC TGC GTG GTG CAC TAC ACC GGG ATG 90

CTT GAA GAT GGA AAG AAA TTT GAT TCC TCC 120

CGG GAC AGA AAC AAG CCC TTT AAG TTT ATG 150

CTA GGC AAG CAG GAG GTG ATC CGA GGC TGG 180

GAA GAA GGG GTT GCC CAG ATG AGT GTG GGT 210

CAG AGA GCC AAA CTG ACT ATA TCT CCA GAT 240

TAT GCC TAT GGT GCC ACT GGG CAC CCA GGC 270

ATC ATC CCA CCA CAT GCC ACT CTC GTC TTC 300

GAT GTG GAG CTT CTA AAA CTG GAA 324

3. An expression vector containing the DNA molecule according to claim 1 or claim 2, wherein said DNA molecule is Introduced into a cloning site downstream from a promoter and upstream from a terminator in the vector.

4. An expression vector according to claim 3 wherein said vector is a plasmid.

5. An expression vector according to claim 4 wherein said vector is pJDB-GAP-FKBP-A (FERM BP-3471).

6. A transformant obtained by transforming a host with the expression vector according to any one of claims 3 to 5.

7. A transformant according to claim 6 wherein said host is yeast or *Escherichia coli.*

8. A process for manufacturing a recombinant human FK506-binding protein that comprises the amino acid sequence according to claim 1, which comprises the steps of:
constructing an expression vector capable of replicating and of expressing a DNA molecule containing the base sequence according to claim 2 in an appropriate host;
incorporating said expression vector into the host to produce transformant cells;
culturing said transformant cells under such conditions that said DNA is expressed to produce said recombinant human FK506-binding protein; and
recovering said recombinant human FK506-binding protein.

9. A process according to claim 8 wherein said vector is pJDB-GAP-FKBP-A (FERM BP-3471) and said host is yeast.

10. A recombinant human FK506-binding protein obtainable by the process of claim 8 or 9 and comprising the amino acid sequence as defined in claim 1.

## Patentansprüche

1. DNA-Molekül, umfassend eine Basensequenz, die für humanes FK506-bindendes Protein codiert, das durch die folgende Aminosäuresequenz dargestellt wird:

1                   5                        10
Met Gly Val Gln Val Glu Thr Ile Ser Pro

                        15                       20
Gly Asp Gly Arg Thr Phe Pro Lys Arg Gly

                        25                       30
Gln Thr Cys Val Val His Tyr Thr Gly Met

                        35                       40
Leu Glu Asp Gly Lys Lys Phe Asp Ser Ser

                        45                       50
Arg Asp Arg Asn Lys Pro Phe Lys Phe Met

                        55                       60
Leu Gly Lys Gln Glu Val Ile Arg Gly Trp

                        65                       70
Glu Glu Gly Val Ala Gln Met Ser Val Gly

                        75                       80
Gln Arg Ala Lys Leu Thr Ile Ser Pro Asp

85 90
Tyr Ala Tyr Gly Ala Thr Gly His Pro Gly

95 100
Ile Ile Pro Pro His Ala Thr Leu Val Phe

105
Asp Val Glu Leu Leu Lys Leu Glu

2. DNA-Molekül nach Anspruch 1, wobei die Basensequenz die folgende Sequenz umfaßt:

ATG GGA GTG CAG GTG GAA ACC ATC TCC CCA 30
GGA GAC GGG CGC ACC TTC CCC AAG CGC GGC 60
CAG ACC TGC GTG GTG CAC TAC ACC GGG ATG 90
CTT GAA GAT GGA AAG AAA TTT GAT TCC TCC 120
CGG GAC AGA AAC AAG CCC TTT AAG TTT ATG 150
CTA GGC AAG CAG GAG GTG ATC CGA GGC TGG 180
GAA GAA GGG GTT GCC CAG ATG AGT GTG GGT 210
CAG AGA GCC AAA CTG ACT ATA TCT CCA GAT 240
TAT GCC TAT GGT GCC ACT GGG CAC CCA GGC 270
ATC ATC CCA CCA CAT GCC ACT CTC GTC TTC 300
GAT GTG GAG CTT CTA AAA CTG GAA 324

3. Expressionsvektor, enthaltend das DNA-Molekül nach Anspruch 1 oder Anspruch 2, wobei das DNA-Molekül in eine Clonierungsstelle stromabwärts von einem Promotor und stromaufwärts von einem Terminator in den Vektor eingeführt ist.

4. Expressionsvektor nach Anspruch 3, wobei der Vektor ein Plasmid ist.

5. Expressionsvektor nach Anspruch 4, wobei der Vektor pJDB-GAP-FKBP-A (FERM BP-3471) ist.

6. Transformant, erhalten durch Transformation eines Wirts mit dem Expressionsvektor nach einem der Ansprüche 3 bis 5.

7. Transformant nach Anspruch 6, wobei der Wirt Hefe oder Escherichia coli ist.

8. Verfahren zur Herstellung eines rekombinanten humanen FK506-bindenden Proteins, das die Aminosäuresequenz gemäß Anspruch 1 umfaßt, wobei das Verfahren folgende Stufen umfaßt:
Konstruktion eines Expressionsvektors, der zur Replikation und Expression eines DNA-Moleküls, das die Basensequenz gemäß Anspruch 2 enthält, in einem geeigneten Wirt imstande ist;
Einfügen des Expressionsvektors in den Wirt zur Bildung von Transformantenzellen;
Kultivieren der Transformantenzellen unter solchen Bedingungen, daß die DNA unter Bildung des rekombinanten humanen FK506-bindenden Proteins exprimiert wird; und
Gewinnung des rekombinanten humanen FK506-bindenden Proteins.

9. Verfahren nach Anspruch 8, wobei der Vektor pJDB-GAP-FKBP-A (FERM BP-3471) ist und der Wirt Hefe ist.

10. Rekombinantes humanes FK506-bindendes Protein, das nach dem Verfahren nach Anspruch 8 oder 9 erhältlich ist und die Aminosäuresequenz gemäß der Definition in Anspruch 1 umfaßt.

## Revendications

1. Molécule d'ADN comprenant une séquence de base codant pour la protéine de liaison au FK506 humain, qui est représentée par la séquence d'acides aminés suivante :

1                   5                   10
Met Gly Val Gln Val Glu Thr Ile Ser Pro

                    15                  20
Gly Asp Gly Arg Thr Phe Pro Lys Arg Gly

                    25                  30
Gln Thr Cys Val Val His Tyr Thr Gly Met

                    35                  40
Leu Glu Asp Gly Lys Lys Phe Asp Ser Ser

                    45                  50
Arg Asp Arg Asn Lys Pro Phe Lys Phe Met

                    55                  60
Leu Gly Lys Gln Glu Val Ile Arg Gly Trp

                    65                  70
Glu Glu Gly Val Ala Gln Met Ser Val Gly

                    75                  80
Gln Arg Ala Lys Leu Thr Ile Ser Pro Asp

                    85                  90
Tyr Ala Tyr Gly Ala Thr Gly His Pro Gly

95                    100
Ile Ile Pro Pro His Ala Thr Leu Val Phe

105
Asp Val Glu Leu Leu Lys Leu Glu

2. Molécule d'ADN selon la revendication 1, dans laquelle ladite séquence de base comprend la séquence suivante :

ATG GGA GTG CAG GTG GAA ACC ATC TCC CCA 30
GGA GAC GGG CGC ACC TTC CCC AAG CGC GGC 60
CAG ACC TGC GTG GTG CAC TAC ACC GGG ATG 90
CTT GAA GAT GGA AAG AAA TTT GAT TCC TCC 120
CGG GAC AGA AAC AAG CCC TTT AAG TTT ATG 150
CTA GGC AAG CAG GAG GTG ATC CGA GGC TGG 180
GAA GAA GGG GTT GCC CAG ATG AGT GTG GGT 210
CAG AGA GCC AAA CTG ACT ATA TCT CCA GAT 240
TAT GCC TAT GGT GCC ACT GGG CAC CCA GGC 270
ATC ATC CCA CCA CAT GCC ACT CTC GTC TTC 300
GAT GTG GAG CTT CTA AAA CTG GAA 324

3. Vecteur d'expression contenant la molécule d'ADN selon la revendication 1 ou la revendication 2, dans lequel ladite molécule d'ADN est introduite dans un site de clonage en aval d'un promoteur et en amont d'un terminateur dans le vecteur.

4. Vecteur d'expression selon la revendication 3, dans lequel ledit vecteur est un plasmide.

5. Vecteur d'expression selon la revendication 4, dans lequel ledit vecteur est pJDB-GAP-FKBP-A (FERM BP-3471).

6. Transformant obtenu en transformant un hôte avec le vecteur d'expression selon l'une quelconque des revendications 3 à 5.

7. Transformant selon la revendication 6, dans lequel ledit hôte est une levure ou *Escherichia coli.*

8. Procédé de fabrication d'une protéine recombinante de liaison au FK506 humain contenant la séquence d'acides aminés selon la revendication 1, comprenant les étapes consistant à :
construire un vecteur d'expression capable de répliquer et d'exprimer une molécule d'ADN contenant la séquence de base selon la revendication 2 dans un hôte approprié ;
incorporer ledit vecteur d'expression dans l'hôte afin de produire des cellules transformées ;
cultiver lesdites cellules transformées dans des conditions telles que ledit ADN est exprimé afin de produire ladite protéine recombinante de liaison au FK506 humain ; et
recueillir ladite protéine recombinante de liaison au FK506 humain.

9. Procédé selon la revendication 8, dans lequel ledit vecteur est pJDB-GAP-FKBP-A (FERM BP-3471) et ledit hôte est une levure.

10. Protéine recombinante de liaison au FK506 humain pouvant être obtenue par le procédé de la revendication 8 ou 9 et comprenant la séquence d'acides aminés telle que définie dans la revendication 1.
